# EUROPÄISCHE PATENTSCHRIFT

(11) **EP 0 767 371 B1**
(45) Veröffentlichungstag und Bekanntmachung des Hinweises auf die Patenterteilung: **05.06.2002**
(21) Anmeldenummer: 96111498.0
(22) Anmeldetag: 17.07.1996
(51) Int. Cl.: G01N 27/22, G01N 33/44

(54) **Verfahren zur Bestimmung spezifischer Materialcharakteristiken**
Process for determining specific material characteristics
Procédé de détermination des caractéristiques spécifiques d'un matériau

(30) Priorität: 02.10.1995 DE 19536766
(43) Veröffentlichungstag der Anmeldung: 09.04.1997
(73) Patentinhaber: SOMOS GmbH, D-64331 Weiterstadt (DE)
(72) Erfinder: Becker, Achim, 64293 Darmstadt (DE); Menzel, Jan, 60318 Frankfurt (DE)
(74) Vertreter: Voth, Gerhard, Dipl.-Ing.

(56) Entgegenhaltungen:
- EP-A- 0 108 629
- EP-A- 0 207 377
- US-A- 4 399 100
- US-A- 4 584 522
- US-A- 4 791 354
- US-A- 4 932 243
- US-A- 5 317 252

## Beschreibung

Die Erfindung betrifft eine Vorrrichtung zur Bestimmung spezifischer Materialcharakteristiken von Kunststoffen nach dem Oberbegriff des Hauptanspruchs.

Eine solche Vorrichtung ist beispielsweise aus der US-PS 45 68 874 bekannt und betrifft das nichtinvasive Messen von Material beispielsweise von Kunststoffmaterial mit einem elektrischen Feld. Bei dem bekannten Verfahren sind drei Elektroden erforderlich, wobei zwei Elektroden als Meßelektroden ausgebildet sind. Eine weitere Elektrode dient zur Abschirmung für die beiden Meßelektroden. Es ist auch bekannt, anstelle einer Elektrode zur Abschirmung eine Nebenschlußelektrode anzuordnen. Mit dieser erzielt man eine Kompensation bestimmter Materialeigenschaften. Ein Nachteil der bekannten Anordnung besteht jedoch darin, daß die Sensortechnik recht aufwendig und mit einem hohen Unsicherheitsfaktor belastet ist.

Aus der EP 0 108 629 ist ein Sensor bekannt, der durch ringförmige koaxiale Kapazitätssensoren gebildet wird, welche ringförmige Spaltungen aufweisen. Durch die koaxiale Anordnung der Kapazitätssensoren wird eine Messung eines Schüttgutes ermöglicht.

Die US PS 5 317 252 beschreibt einen frequenzabhängigen elektromagnetischen Sensor.

Der Erfindung liegt daher die Aufgabe zugrunde eine Vorrichtung zur Bestimmung spezifischer Materialcharakteristiken von Kunststoffen zu schaffen, welche genaue Aussagen zuläßt und einen geringen konstruktiven Aufwand erfordert.

Diese Aufgabe wird ausgehend von dem Oberbegriff des Hauptanspruchs durch dessen kennzeichnenden Merkmale gelöst.

Der wesentliche Vorteil der Endung besteht darin, daß ein kapazitiver Sensor mit Wechselspannung beaufschlagt wird und die gemessene Impedanz unmittelbar die Bezugsgröße für eine bestimmte Materialcharakteristik darstellt.

Eine Ausgestaltung des Sensors sieht vor, diesen aus wenigstens zwei zueinander konisch verlaufenden Platten aufzubauen. Die Platten sind trichterförmig angeordnet. Dies hat den Vorteil, daß das Material, sofern es sich um Schüttgut handelt, innnerhalb des derart gebildeten Trichters eine normierte Schüttgutdichte aufweist. Selbstverständlich kann der Sensor auch andere Formen aufweisen, wie zum Beispiel zwei parallel verlaufende Platten, ringförmig angeordnete Platten oder parallel verlaufende Leiterelemente.

Aus dem gemessenen Wert wird in einer bevorzugten Auswertung eine Vielzahl von Materialcharakteristiken bestimmt. So kann beispielsweise die Zusammensetzung des Materials, der Molekularzustand, das heißt, ob sich das Material im kristallinen oder amorphen Zustand befindet, der Trocknungsgrad, das heißt die im Material noch enthaltene Feuchte, die Produkttemperatur aber auch die Massenübergangszone, beispielsweise in Zeolithen ermittelt werden. Außerdem kann die Schüttgutdichte oder das Mischungsverhältnis unterschiedlicher Materialien zum Beispiel das Mischungsverhältnis von neuem Material zu Recyclingmaterial ermittelt werden.

Diese und weitere Merkmale von bevorzugten Weiterbildungen der Erfindung gehen außer aus den Ansprüchen auch aus der Beschreibung und den Zeichnungen hervor.

Ausführungsbeispiele der Erfindung sind in den Zeichnungen dargestellt und werden im folgenden näher erläutert. Die Zeichnungen zeigen:
- Figur 1: ein Trockner für Kunststoffgranulat, mit einer Einrichtung zur Bestimmung von Materialcharakteristiken,
- Figur 2: Signalverlauf Temperatur,
- Figur 3: Signalverlauf Trocknung,
- Figur 4: Fingerabdrücke (Fingerprints) von Kunststoffgranulat,
- Figur 5: Signalverlauf bei Zeolith,
- Figur 6: ein kapazitiver Sensor.

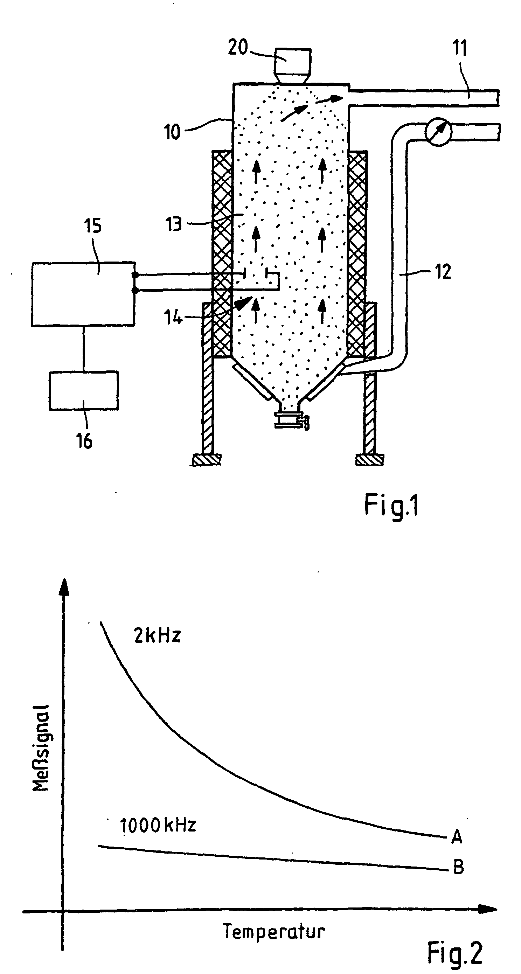

In Figur 1 ist ein Trockner für Kunststoffgranulat gezeigt. Dieser besteht im wesentlichen aus einem Behälter 10, welcher über eine Fülleinrichtung 20 mit Granulat kontinuierlich befüllt wird. Dem Behälter wird über die Leitung 12 trockene, erwärmte Luft zugeführt. Diese Luft strömt durch das im Behälter befindliche Kunststoffgranulat 13 und über die Leitung 11 zu einem hier nicht dargestellten Lufttrocknungssystem. Im Behälter 10 ist ein Sensor in Form eines Plattensensors 14 angeordnet. Dieser ist an eine Auswerteeinheit 15 angeschlossen. Die Auswerteeinheit ist mit einem Display 16 verbunden. Durch die Auswerteeinheit 15 wird der Sensor mit einer Wechselspannung innerhalb eines bestimmten Frequenzspektrums oder mit einer bestimmten Frequenz versorgt. Die gemessene Kapazität ist ein Maß für eine bestimmte Materialcharakteristik des Schüttguts bzw. des Kunststoffgranulats. Durch Aufprägen definiert unterschiedlicher, elektrischer Wechselfelder auf ein Material und Messen der elektrischen Impedanz lassen sich eindeutig momentane Zustandsgrößen bestimmen. Das Verfahren beruht darauf, daß Material spezifische Kenngrößen der einzelnen Komponenten in speziellen Wechselfeldbereichen besonders gut darstellbar sind.

Figur 2 zeigt einen typischen Signalverlauf. Die materialcharakteristische Temperaturabhängigkeit des Kunststoffgranulats wird durch Verändern der Temperatur des Granulats und gleichzeitigem Aufnehmen der Signalmeßdaten ermittelt. Die optimale Meßfrequenz wird so gewählt, daß eine Temperaturänderung eine größtmögliche Signaländerung bewirkt. Es handelt sich bei der Temperaturmessung nicht um eine Punktmessung. Es wird die Mischtemperatur der sich im Sensor befindlichen Granulatelemente ermittelt. Die Figur 2 zeigt, daß bei einer geringeren Temperatur das Meßsignal A mit einer Frequenz von 2 Kilohertz, eine hohen Pegel aufweist, der sich mit Erhöhung der Temperatur verringert. Beträgt die Frequenz des Meßsignals 1000 Kilohertz, ist die Änderung über einen bestimmten Temperaturbereich geringer ausgeprägt. Es ist deshalb zweckmäßig, zur Ermittlung der Granulattemperatur das Signal A auszuwerten.

Mit dem Verfahren kann ebenso die Feuchtemessung von hygroskopischem Material ermittelt werden. Hygroskopisches Material dient zum Aufnehmen von Feuchtigkeit, die beispielsweise bei dem Trocknen von Kunststoffgranulat entsteht. Das hygroskopische Material wird befeuchtet, indem es mit feuchter Luft durchströmt wird. Ein Sensor der sich im Materialstrom befindet, wird mit einem breiten elektrischen Frequenzband beaufschlagt. Eine Rechnereinheit nimmt die Meßdaten auf. Die Meßfrequenz wird derart gewählt, daß eine Feuchtigkeitsveränderung eine größtmögliche Signalveränderung zur Folge hat. Diese anzustrebende Meßfrequenz hängt vom Materialtyp und der Temperatur ab. Die erstellte Materialkennlinie wird in eine Rechnereinheit abgelegt und stellt den Zusammenhang zwischen Feuchtigkeit und elektrischem Meßsignal her.

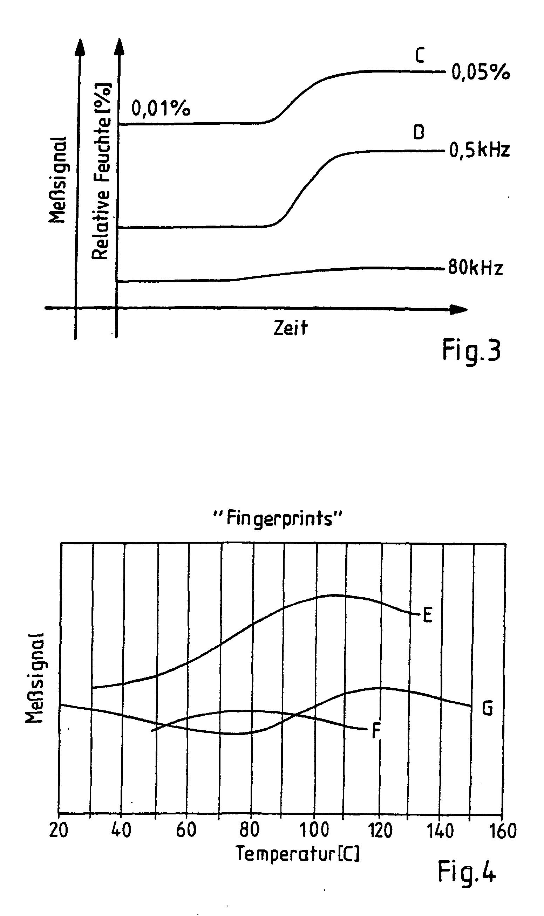

Das Diagramm gemäß Figur 3 zeigt mit Signalverlauf C den Zusammenhang zwischen Meßsignal und relativer Feuchte bei dem Anstieg der relativen Feuchtigkeit von 0,01 % auf 0.05 %. Das Meßsignal welches bei einer Frequenz von 80 Kilohertz aufgenommen wurde (Kurve D) zeigt bei dem Anstieg der relativen Feuchte einen Amplitudenvergrößerung welche analog zu dem Anstieg der relativen Feuchte verläuft und damit ein Maß für die Beladung des hygroskopischen Materials mit Feuchtigkeit darstellt.

Figur 4 zeigt die Meßkurven verschiedener Kunststoff in einem sogenannten Fingerprint. Diese Meßkurven wurden über eine Temperaturveränderung im Bereich von 20 bis 160 ° aufgenommen. Verlauf E zeigt ein Polyamid, Verlauf F Co-Polyester und Verlauf G einen Polyäthylen-Kunststoff. Selbstverständlich besteht auch die Möglichkeit, verschiedene Materialien mit bestimmten Mischungsverhältnissen als sogenannte Fingerprints abzuspeichern und das sensierte Material mit diesen Daten zu vergleichen. Damit läßt sich das Mischungsverhältnis feststellen.

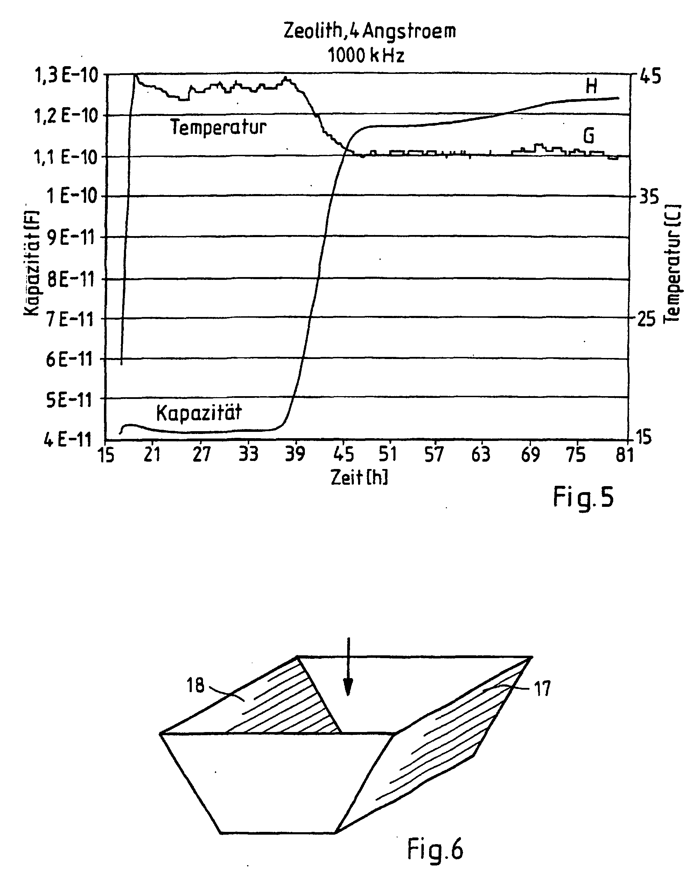

Figur 5 zeigt den Zusammenhang zwischen der Materialtemperatur und des Kapazitätssignals. Während die Temperatur (Verlauf G) von einem bestimmten Punkt aus innerhalb eines Zeitraums abgesenkt wurde, steigt die Kapazität innerhalb dieses Bereichs sehr stark an. Damit ist die Kapazität unmittelbar ein Signal für die zu messende Temperatur.

Figur 6 zeigt einen Sensor aus zwei zueinander konisch verlaufenden Platten 17. 18. Durch diesen Sensor wird von oben her das zu sensierende Kunststoffgranulat geführt. Aufgrund der gegenüber der Eintrittsfläche verringerte Austrittsfläche bildet sich ein gewisser Materialstau der zu einer Nomierung der Schüttgutdichte innerhalb dieses Sensors führt.

## Patentansprüche

1. Einrichtung zur Bestimmung spezifischer Materialcharakteristiken von Kunststoffen, aufweisend
• einen kapazitiven Sensor der mit Wechselspannung wenigstens einer bestimmten Frequenz beaufschlagt wird und dessen gemessene Impedanz eine Bezugsgröße für eine bestimmte Materialcharakteristik darstellt, wobei der Sensor im wesentlichen aus zwei beabstandet angeordneten elektrischen Leitern besteht, zwischen welchen sich das zu analysierende Material befindet,
• eine Einrichtung zur Erzeugung eines Wechselspannungssignals,
• eine weitere Einrichtung zur Auswertung der Kapazitätsgröße oder des Leitwertes
• eine weitere Einrichtung zum Vergleich des ermittelten Wertes mit vorgegebenen Kenngrößen,
**dadurch gekennzeichnet, daß** die zwei beabstandet angeordneten elektrischen Leitern des Sensors aus zwei zueinander konisch verlaufenden Platten (17, 18) bestehen, welche derart angeordnet sind, daß eine Normierung der Schüttgutdichte innerhalb des Sensors erfolgt.

2. Einrichtung nach Anspruch 1, **dadurch gekennzeichnet, daß** aus der gemessenen lmpedanz mittels eines abgespeicherten Vergleichswertes in einer Rechnereinheit die Materialart und/oder die Zusammensetzung des Materials und/oder der Molekularzustand und/oder der Trocknungsgrad und/oder die Durchschnittsprodukttemperatur und/oder die Massenübergangszone und/oder die Schüttdichte und/oder das Mischungsverhältnis unterschiedlicher Materialien bestimmt wird.

3. Einrichtung nach Anspruch 1 oder 2, **dadurch gekennzeichnet, daß** die Impedanz während einer Temperaturänderung bestimmt wird.

4. Einrichtung nach Anspruch 3, **dadurch gekennzeichnet, daß** die Temperaturänderung kontinuierlich oder in Stufen erfolgt.

## Claims

1. Apparatus for determining specific material characteristics of plastics materials, including
• a capacitive sensor, to which an alternating voltage at least of a predetermined frequency is applied, the measured impedance of which sensor represents a reference parameter for a predetermined material characteristic, the sensor substantially comprising two spaced-apart electrical conductors, between which is situated the material to be analysed,
• an apparatus for generating an alternating voltage signal,
• an additional apparatus for evaluating the magnitude of capacitance or the conductance,
• an additional apparatus for comparing the ascertained value with prescribed characteristic magnitudes,
**characterised in that** the two spaced-apart electrical conductors of the sensor comprise two plates (17, 18), which taper towards each other and are disposed in such a manner that the loose material density within the sensor is normalised.

2. Apparatus according to claim 1, **characterised in that** the type of material and/or the composition of the material and/or the molecular state and/or the degree of dryness and/or the mean product temperature and/or the mass transition zone and/or the loose density and/or the mixture ratio between different materials are/is determined from the measured impedance by means of a stored comparison value in a computer unit.

3. Apparatus according to claim 1 or 2, **characterised in that** the impedance is determined during a change in temperature.

4. Apparatus according to claim 3, **characterised in that** the change in temperature occurs continuously or in stages.

## Revendications

1. Installation pour déterminer les caractéristiques spécifiques de matériau pour des matières plastiques comprenant
- un capteur capacitif recevant une tension alternative d'au moins une certaine fréquence et dont l'impédance mesurée représente une grandeur de référence pour une certaine caractéristique de matériau, le capteur étant formé principalement de deux conducteurs électriques distincts l'un de l'autre, et entre lesquels se trouve la matière à analyser,
- une installation pour générer un signal de tension alternative,
- une autre installation pour exploiter la capacité ou la conduction et
- une autre installation pour comparer la valeur obtenue à des grandeurs caractéristiques prédéterminées,
**caractérisée en ce que**
les deux conducteurs électriques du capteur, écartés, sont formés par deux plaques (17, 18) qui se rapprochent en cône et sont installées pour produire une densité du produit en vrac normalisé dans le capteur.

2. Installation selon la revendication 1,
**caractérisée en ce qu'**
à partir de l'impédance mesurée, à l'aide d'une valeur de comparaison mémorisée dans une unité de calcul, on détermine le type de matière et/ou la composition de la matière et/ou l'état moléculaire et/ou le degré de dessiccation et/ou la température moyenne du produit et/ou la zone de transition de masse et/ou la densité en vrac et/ou le rapport de mélange de matières différentes.

3. Installation selon l'une quelconque des revendications 1 ou 2,
**caractérisée en ce qu'**
on détermine l'impédance pendant une variation de température.

4. Installation selon la revendication 3,
**caractérisée en ce que**
la variation de température est continue ou par gradins.
